# EUROPEAN PATENT SPECIFICATION

(11) **EP 1 711 148 B1**
(45) Date of publication and mention of the grant of the patent: **08.02.2012**
(21) Application number: 04801713.1
(22) Date of filing: 03.12.2004
(51) Int. Cl.: D04H 1/70, A61F 13/15

(54) **APPARATUS AND METHOD FOR MANUFACTURING AN ABSORBENT CORE**
GERÄT UND VERFAHREN ZUR HERSTELLUNG EINES SAUGFÄHIGEN KERNS
APPAREIL ET PROCEDE DE FABRICATION D'UN NOYAU ABSORBANT

(30) Priority: 02.02.2004 SE 0400201
(43) Date of publication of application: 18.10.2006
(73) Proprietor: SCA HYGIENE PRODUCTS AB, 405 03 Göteborg (SE)
(72) Inventor: FORSBRING, Göran, S-434 47 Kungsbacka (SE); LARSSON, Gunnar, S-438 32 Landvetter (SE)
(74) Representative: VALEA AB
(86) International application number: PCT/SE2004/001796
(87) International publication number: WO 2005/072671

(56) References cited:
- EP-A1- 0 297 180
- EP-A1- 0 627 211
- WO-A2-03/053297
- US-B1- 6 652 798

## Description

### Technical field

The present invention relates to a method and an apparatus for the manufacturing of an absorbent core containing particles of a second material, such as super absorbent polymers (SAP), dispersed within a first absorbent material. More specifically, the present invention is directed to the manufacture of an absorbent core with the object of reduction of losses of the second material during manufacture.

### Background of the invention

Absorbent products, such as diapers, training pants, napkins, incontinence pads and the like, contains an absorbent core usually comprising a soft, fluffy material, such as comminuted wood pulp fibers. Various secondary materials are often incorporated into the absorbent fluff material, such as particles of superabsorbent polymers (SAP), heat activatable bonding fibers or odour absorbent material. Super absorbent materials are polymers having the ability to absorb water and bodily fluids many times there own weight. The super absorbent material is either mixed with the fluff pulp fibers or applied in a layered configuration between layers of fluff pulp.

One way to produce such an absorbent core containing a second material is to form a first layer of absorbent fluff pulp, whereupon the second material is sprinkled on top of the fluff pulp. A second layer of absorbent fluff pulp is then placed on top of the second material to complete the core. The absorbent core may further comprise two or more layers of the second material disposed between layers of absorbent fluff pulp fibers. A result of this method is that it produces a product with a layered configuration, in which the second material is concentrated in fairly discrete zones within the core.

Alternatively, the second material (such as SAP particles) is mixed with and distributed throughout the first material (the absorbent fluff pulp fibres). It is further known to make absorbent products including a first and a second layer, in which the first layer contains a pure first material and the second layer contains a mixture of the first and the second material.

One way to produce absorbent core for products with a fairly uniform distribution of the second material within the core is disclosed in patent document US 5 447 677. All statements and drawings in said document are hereby included, by reference, in this description. In said disclosure there is presented an apparatus for making absorbent products containing a first material, such as absorbent fluff from wood pulp fibres. Said absorbent fluff pulp is introduced into a vacuum-forming chamber. A portion of the fluff pulp is deposited into the cavity of a mold transported through the forming chamber by a forming wheel so as to form a layer of pure fluff pulp within the bottom of the mold cavity. A second material, such as superabsorbent particles or heat activatable bonding fibres, is introduced into the forming chamber so that streams of the first and second materials collide within a mixing zone. As the mold continues it travels through the forming chamber. A mixture of first and second materials from the mixing zone is deposited within the mold cavity, thereby filling it. The result is an absorbent core having a first layer formed by pure first material and a second layer formed by a mixture of the first and second materials.

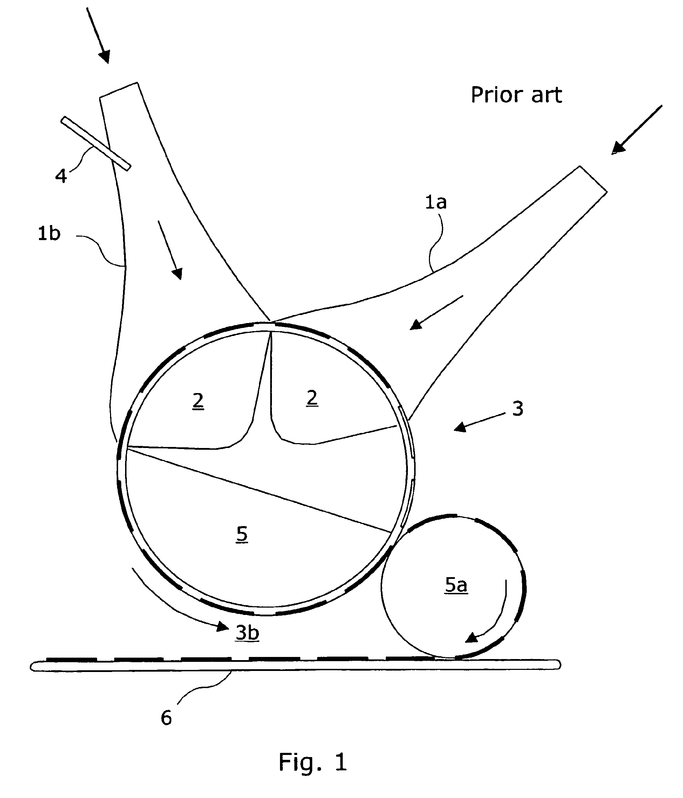

A more detailed description of prior art manufacture of an absorbent product of the design as discussed is made here by reference to figure 1. From a defibration unit pulp fibres are transported by the aid of air up to hoods 1a, 1b and through the hoods towards a forming wheel 3. The forming wheel 3 is provided with stationary suction boxes 2, wherein very low pressure prevails. The suction boxes face said hoods containing the pulp fibres. On the surface of the forming wheel 3 an air pervious means, such as a net or a perforated plate, is provided. When said air pervious means rotates, in the direction shown by the arrow 3b, under said hoods the pulp fibres are forced by an air stream towards the air pervious means and form a core of pulp fibres along the circumference of the forming wheel in the shape of a continuous mat. Said circumference may be provided with molds for forming separate absorbent cores of different designs.

The core may further be formed into several layers by the use of several hoods. The core may, as stated, be formed into discrete cores or if a continuous mat-formed core is formed, it may be later cut into the desired design.

A flow of SAP-fibres is added to the pulp fibres by an injection of the SAP-fibres through a pipe 4 into one or more of the hoods 1. After the last hood (in the figure hood 1b) the core formation is completed and the core is kept in the mold or molds on the wheel by means of an underpressure in a second stationary suction box 5. The core is, after the passage past said second suction box 5, transferred to a transfer drum 5a, where it may be compressed, for example by means of a mechanical pressure exerted on the core during its passage over the transfer drum. Finally, the core is transferred to a conveyor 6 for further treatment or packaging. Transfer from the forming wheel 3 to the conveyor 6 may be to undertaken without the transfer drum.

An end product may consist of several cores, whereby the manufacturing assembly for said end product consists of two or more forming wheels 3, each one of the wheels forming a core, whereupon the two or more resulting cores are assembled to the end product.

A disadvantage in the discussed manufacturing process is that SAP-particles or fibres are easily lost from the core after the completion of the core formation on the forming wheel 3 along the distance the core travels until it is transferred to the conveyor. Even if a very high vacuum is utilized in the second suction box 5, it is very difficult to retain all of the SAP-particles in the formed core, especially if high speeds are used for the transporters, that is, the conveyor, the forming wheel and the transfer drum. Arrangements may be made to collect the lost SAP-particles in funnels. In this way, portions of the lost amount of SAP-particles are collected and may be returned to the core forming process. Other portions of the lost SAP-particles must be discarded. Still other portions of said particles land on the machinery, whereby the machinery has to be cleaned regularly. SAP-particles may also fall down on an underlying conveyor, which results in a product having SAP-particles in undesired locations of the core. Not only SAP-particles are lost during the transfer of the completed core to the conveyor, but also pulp fibres may escape during said transport of the core.

Another way to try to solve the indicated disadvantage would be to increase the vacuum inside the second suction box 5. This could cause the SAP-particles to penetrate deeper into the underlying first layer of pulp fibres and even to be sucked out of the core and clog the air pervious means or to escape into the second suction box 5 and cause problems in the vacuum generation equipment.

### Prior art documents:

EP0297180, EP0627211, US 6652798, WO03053297 further disclose apparatus and methods of manufacturing absorbent cores.

The present invention discloses a way to improve the manufacturing process and to provide measures to overcome the indicated disadvantages.

### SUMMARY OF THE INVENTION

According to a first aspect of the present invention there is provided an apparatus for manufacturing an absorbent core for an absorbent product containing a first material and a second material dispersed throughout at least a portion of said first material, wherein the apparatus comprises:
- a rotating forming wheel provided with air pervious means along its circumferential surface,
- at least one forming chamber along a first arc of said surface for receiving fibres of said first material and means for directing said fibres of said first material in a stream towards said surface of the forming wheel for depositing a layer of said fibres on said surface,
- at least one of said forming chambers having an inlet for introduction of particles of said second material into said stream for depositing a layer of a mixture of said fibres and said particles on the surface of said wheel,
and that
an air pervious belt is provided along a second arc of the circumferential surface of the forming wheel, wherein said belt bears on the formed at least one layer of said first and second materials and runs at the same speed as the surface of the forming wheel for keeping the fibres and particles of said formed layer or layers in place on the wheel. The circumferential surface of the forming wheel is provided with a continuous mold or a number of separate molds along the surface.

According to a second aspect of the invention there is provided a method for manufacturing an absorbent product containing a first material and a second material dispersed throughout at least a portion of said first material, wherein the method comprises the steps of:
- providing a forming wheel having air pervious means along its circumferential surface,
- rotating said forming wheel,
- arranging at least one forming chamber along a first arc of said surface,
- receiving in said forming chamber fibres from said first material,
- directing said fibres of said first material in a stream towards said surface of the forming wheel,
- introducing in at least one of said forming chambers particles of said second material into said stream,
- depositing at least a layer of said first and said second materials on said surface,
- providing at least one mold along said circumferential surface,
- covering said mold along a second arc of the forming wheel with a belt running at substantially the same speed as the forming wheel at the surface of said wheel.

In short, the apparatus is provided with a continuous air pervious belt, which runs together with the forming wheel, substantially at the same speed as the forming wheel at the surface of the forming wheel, where the main purpose of the belt is to cover the continuous layer or molds arranged for the formation of an absorbent core on the circumferential surface of said wheel, such that pulp fibres and particles of the second material are unable to fall out of the core.

An advantage with the various aspects of the inventions is that loss of material will be reduced and particularly loss of the second material will be reduced. Another advantage is that an air flow radially inwards through the air pervious means provided to keep the absorbent core and its fibres and particles within the mold may be reduced as the core is covered with the inventive belt. Further, the underpressure in the second suction box along the arc of the forming wheel where there are no forming chambers will be enforced, which in turn further contributes to better keep the fibres and particles of the layers of the core in place during transport of the core on the surface of the forming wheel. Due to the reduced air flow, the power consumption of the fan related to the second suction box will be reduced. This reduction in power consumption is also valid for production of absorbent cores where there is no addition of particles of a second material. Further, as the loss of fibres and particles is reduced in comparison to prior art design devices, stoppages in production for cleaning of the machinery may be reduced.

Still further objects and features of the present invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not necessarily drawn to scale and that, unless otherwise indicated, they are merely intended to conceptually illustrate the structures and procedures described herein.

### BRIEF DESCRIPTION OF THE DRAWINGS

Fig. 1 schematically shows an absorbent core-manufacturing apparatus according to prior art.
Fig. 2 schematically shows en embodiment of the manufacturing apparatus according to one aspect of the invention.

### EMBODIMENTS OF THE INVENTION

A number of embodiments of the invention will be presented in the following with support of the enclosed figures.

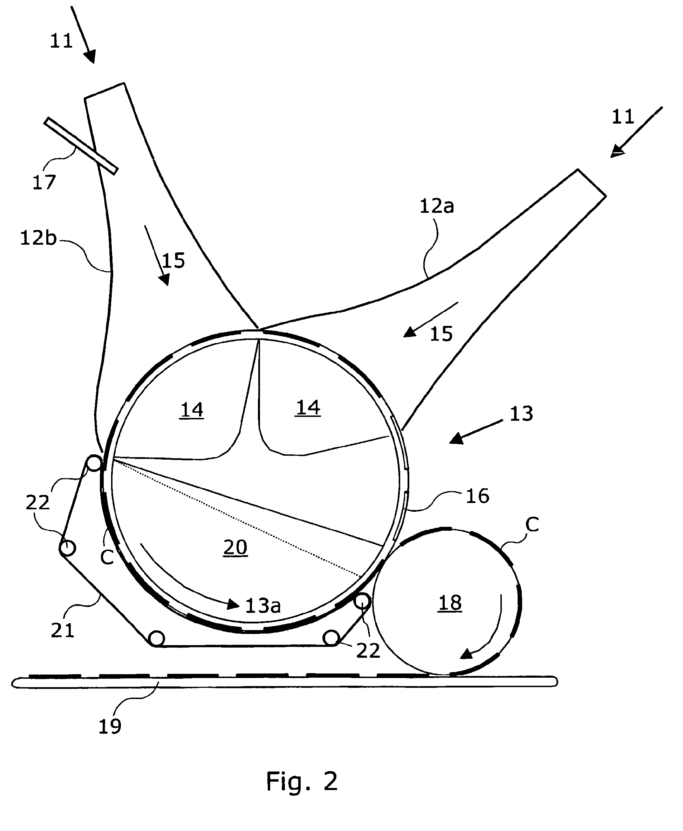

In figure 2, there is disclosed an apparatus for manufacturing an absorbent core of a liquid-absorbent product according to various aspects of the invention. From a defibration unit (not shown) pulp fibres 11 are delivered by the aid of an air stream into forming chambers 12a, 12b, here called hoods. A forming wheel 13 for formation of a core of an absorbent product is arranged to rotate under a downstream opening of a hood 12a, 12b and is in sealed connection with said hood. The forming wheel 13 rotates in the direction shown by arrow 13a. In the embodiment according to the figure, the device is provided with two hoods 12a and 12b. Facing each hood opening, non-rotating first suction boxes 14 are arranged inside the forming wheel 13. An air pervious means is arranged along the circumference of the forming wheel 13 to allow air from said suction boxes 14 to pass radially inwards of the forming wheel. An underpressure prevails in the first suction boxes 14 for sucking air from said hoods 12 through the air pervious means to generate a stream 15 of said radially inwards passing air. Said underpressure may be generated by means of not shown fans.

Fibres delivered to the hoods 12a, 12b are forwarded by the stream 15 towards the circumference of the forming wheel 13 and are deposited there against the air pervious means, which may consist of any net, a plate provided by holes, a fabric or the like.

The forming wheel 13 may be provided with a continuous mold extending along the circumference of the forming wheel. In the figure there is shown an example where a series of separate molds 16 are arranged along the circumference of the forming wheel 13. A continuous mold is utilized when the fibres deposited on the air pervious means are to be used to form a continuous layer, which is subsequently cut into suitable dimensions and shapes to form individual absorbent cores of an absorbent product. Separate molds are used to directly form cores of a special shape and dimension to form an absorbent core of an absorbent product. Variants of molds are known in the art and need not further be discussed herein.

Often the pulp fibres are combined with other fibres or particles with special properties as added ingredients to form the absorbent cores of absorbent products. Figure 2 gives an example of a way to add such particles, which for example may be superabsorbent particles or heat activatable bonding particles or odour absorbent particles. Super absorbent particles are described, inter alia, in US 4 540 454. In the hood 12a pulp fibres 11, only, are used for forming a first layer of the produced core C in the molds 16. A second hood 12b is provided with an inlet 17, through which the particles to be added are injected to be mixed with the pulp fibres 11 and for being added to the stream 15 to be directed as a mixture of pulp fibres 11 and added particles towards the molds 16 on the forming wheel 13. Hence, as the first formed layer rotates from the first hood 12a and enters the second hood 12b, the mixture of pulp fibres and the added particles forms a second layer on top of the first layer to build up the core of the absorbent product. Instead of injecting the added particles into a hood 12 b by means of inlet 17, said hood 12b may be provided with an upstream prepared mixture of pulp fibers 11 and said particles.

According to the prior art technique, as discussed, the completed core in the molds 16 will be transported on the surface of the wheel 13 to a transfer drum 18 and finally to a conveyor belt 19. The transfer drum 18 may be excluded. To keep the core of fibres adhered to the surface of the forming wheel 13, said wheel is provided with a non rotating second suction box 20 inside the wheel 13 and exerting a suction effect on the core, such that it will affix to the surface of the wheel. Said second suction box 20 acts along the surface of the forming wheel from the last one of the forming chambers, the hoods 12a, 12b and as far as the core is transported on the wheel 13. In an alternative embodiment the suction box 20 is not acting along the full second arc of the forming wheel covered by said belt, so that it will be easier to let the core leave the forming wheel (13), when said core is delivered to a transfer drum or a conveyor belt (19), without being sucked to the surface. This alternative embodiment is illustrated by means of the extension of the second suction box being illustrated as a dotted line in figure 2.

To prevent fibres, particularly the added particles, from falling out of, or escaping from the formed core of layers, the manufacturing apparatus is provided with a belt 21 arranged in such a way that the belt 21 exerts a pressure against the forming wheel 13 and in particular against the core with the layers of pulp fibres and added particles. By means of this belt 21, the continuous mold or separate molds 16 are covered by said belt and pulp fibres or added particles are prevented from falling out of the mold during delivery of the completed core to the conveyor belt 19 or transfer drum 18.

As is shown in the example according to the figure, the belt 21 is arranged in a closed, endless loop. The belt 21 is mounted on supporting rolls 22 to allow said loop movement. At least one of said supporting rolls 22 is resiliently mounted, making it possible to stretch the belt, so that it will exert a predetermined pressure force against the forming wheel 13.

The belt 21 is preferably air pervious, so that the air sucked radially inwards to the wheel 13 can easily penetrate the belt. The belt is further laterally wide enough to cover the molds 16 in the lateral direction of the forming wheel 13.

The speed of the belt is preferably adapted to be the same as the speed of the circumference of the forming wheel 13, which is self-accomplished as the belt may be driven by the friction forces generated between the belt 21 and the surface of the forming wheel 13, provided that the belt 21 is moving without too much friction in its loop arrangement. If there is too much friction of the belt movement in the belt loop arrangement, the belt and the forming wheel may slide in relation to each other. The belt movement may of course be arranged in such a way that the belt is driven by the forming wheel or by a driving motor, wherein in both cases this could involve a driving of the belt 21 via a gear arrangement.

## Claims

1. An apparatus for manufacturing an absorbent product containing a first material and a second material dispersed throughout at least a portion of said first material, comprising,
- a rotating forming wheel (13) provided with air pervious means along its circumferential surface,
- means for directing at least a mixture of fibres of said first material and particles of said second material towards said surface of said forming wheel (13) for depositing at least a layer of a mixture of said fibres and said particles along a first arc of said surface of said forming wheel (13),
**characterized in that**
an air pervious belt (21) is provided along a second arc of the circumferential surface of the forming wheel (13), wherein said belt (21) bears on the formed at least one layer of said first and second materials and runs at the same speed as the surface of the forming wheel (13) for keeping the fibres and particles of said formed layer or layers in place on the forming wheel (13),
wherein said means comprises:
- at least one forming chamber (12a, 12b) along said first arc of said surface for receiving at least the fibres of said first material and suction means for directing the fibres of said first material in a stream (15) towards said surface of the forming wheel (13) for depositing a layer of said fibres on said surface and at least one of said forming chambers (12a, 12b) being provided with a mixture of the fibres of said first material and the particles of said second material, and
wherein the circumferential surface of the forming wheel (13) is provided with a continuous mold (16) or a number of separate molds (16) along the surface.

2. The apparatus according to claim 1, wherein the at least one forming chamber (12a,12b) being provided with both the fibres of said first material and the particles of the second material has a separate inlet (17) for the introduction of the particles of said second material into said stream (15).

3. The apparatus according to claim 1 or 2, wherein said suction means for directing the fibres towards the forming wheel (13) comprises at least one non-rotating first suction box (14) inside the circumference of the forming wheel (13), said at least one suction box (14) facing said forming chamber (12a, 12b) and being provided with an underpressure for generating said stream (15) for sucking the fibres from the forming chamber (12a, 12b) in the direction of the air pervious means on the forming wheel (13).

4. The apparatus according to claim 3, wherein a first suction box (14) is provided for each forming chamber (12a, 12b).

5. The apparatus according to any of the preceding claims, wherein the first material is pulp fibres.

6. The apparatus according to any of the preceding claims, wherein the particles of the second material are particles or fibres of superabsorbent polymer or heat activatable bonding material.

7. The apparatus according to any of the preceding claims, wherein said air pervious means includes at least one member from the group of: a net, a plate provided with holes, an air pervious fabric.

8. The apparatus according to any of the preceding claims, wherein a non-rotating second suction box (20) is provided inside the circumference of the forming wheel (13), said second suction box (20) being arranged along said second arc of the circumference of the forming wheel (13) and being provided with an underpressure for sucking the formed layer or layers against the air pervious means on the surface of the forming wheel (13).

9. The apparatus according to claim 8, wherein said belt (21) is arranged to face said second suction box (20).

10. The apparatus according to claim 9, wherein said suction box (20) extends only a portion of the second arc of the forming wheel (13) covered with the belt

11. The apparatus according to any of the preceding claims, wherein said belt (21) is an endless belt running in a closed loop and supported by means of supporting rolls (22).

12. The apparatus according to claim 11, wherein at least one of said rolls (22) is arranged to exert a resilient force on said belt (21) to stretch the belt so that the belt (21) will press against the forming wheel (13) with a predetermined force.

13. The apparatus according to any of claims 8 to 12, wherein the belt (21) has a width broad enough to cover the molds (16) of the forming wheel (13) in a lateral direction.

14. Use of the apparatus according to any of the preceding claims for making an absorbent core (C) in an absorbent product from the group of: sanitary napkin, diaper, training pant, incontinence pad.

15. A method for manufacturing an absorbent structure containing a first material and a second material dispersed throughout at least a portion of said first material using the apparatus as claimed in any one of claims 1 to 13, comprising the steps of:
- providing a forming wheel (13) having air pervious means along its circumferential surface,
- rotating said forming wheel (13),
- arranging at least one forming chamber (12a, 12b) along a first arc of said surface,
- receiving in said forming chamber (12a, 12b) fibres of said first material,
- directing said fibres of said first material in a stream (15) towards said surface of the forming wheel (13),
- introducing in at least one of said forming chambers (12a, 12b) particles of said second material into said stream (15),
- depositing at least a layer of said first and said second materials on said surface,
- providing at least one mold (16) along said circumferential surface,
- covering said mold (16) along a second arc of the forming wheel (13) with a belt (21) running at substantially the same speed as the forming wheel (13) at the surface of said wheel.

16. The method according to claim 15 further comprising the step of:
- arranging said belt (21) to be air pervious.

17. The method according to claims 15 or 16 further comprising the step of:
- arranging said belt (21) to run in a closed loop.

18. The method according to any of claims 15 to 17 further comprising the step of:
- stretching said belt (21) such that it exerts a pressure against the layer or layers of the first and second material in said at least one mold (16) on said surface.

19. Use of the apparatus according to any of claims 1 to 13 for the manufacture of an absorbent core (C) in an absorbent product, said absorbent core consisting of more than one layer, wherein the apparatus comprises a number of forming wheels (13) for the production of at least one layer per forming wheel (13) and wherein each forming wheel (13) is provided with said belt (21).

## Patentansprüche

1. Vorrichtung zum Herstellen eines absorbierenden Produkts, dass ein erstes Material enthält und ein zweites Material, das zumindest innerhalb eines Abschnitts des ersten Materials verteilt ist, mit
- einem rotierenden Formrad (13), das entlang seiner Umfangsfläche mit einem luftdurchlässigen Mittel bereitgestellt ist,
- einem Mittel zum Leiten von zumindest einer Mischung von Fasern des ersten Materials und Partikeln des zweiten Materials in Richtung der Fläche des Formrads (13) zum Ablegen von zumindest einer Lage einer Mischung der Fasern und der Partikel entlang eines ersten Kreisbogens der Oberfläche des Formrads (13),
**dadurch gekennzeichnet, dass**
ein luftdurchlässiges Band (21) entlang eines zweiten Kreisbogens der Umfangsfläche des Formrads (13) bereitgestellt ist, wobei das Band (21) auf der mindestens einen Lage des ersten und zweiten Materials unterstützt und mit der gleichen Geschwindigkeit wie die Fläche des Formrads (13) läuft, um die Fasern und Partikel der gebildeten Lage oder der gebildeten Lagen auf dem Formrad (13) an ihrem Ort zu lassen,
wobei das Mittel aufweist:
- mindestens eine Formkammer (12a, 12b) entlang des ersten Kreisbogens der Flächezum Aufnehmenmindestens der Fasern des ersten Materials und ein Saugmittel zum Leiten der Fasern des ersten Materials in einem Strom (15) in Richtung der Fläche des Formrads (13) zum Ablegen einer Lage der Fasern auf der Oberfläche, wobei mindestens eine der Formkammern (12a, 12b) mit einer Mischung der Fasern des ersten Materials und den Partikeln des zweiten Materials bereitgestellt ist, und wobei die Umfangsfläche des Formrads (13) mit einer durchgehenden Form (16) oder einer Anzahl von getrennten Formen (16) entlang der Fläche bereitgestellt ist.

2. Vorrichtung gemäß Anspruch 1, bei der die mindestens eine Formkammer (12a, 12b), die sowohl mit den Fasern des ersten Materials als auch mit den Partikeln des zweiten Materials bereitgestellt ist, einen getrennten Einlass (17) für das Einführen der Partikel des zweiten Materials in den Strom (15) aufweist.

3. Vorrichtung gemäß Anspruch 1 oder 2, bei der das Saugmittel zum Leiten der Fasern in Richtung des Formrads (13) mindestens eine nicht rotierende erste Saugbox (14) innerhalb des Umfangs des Formrads (13) aufweist, wobei die mindestens eine Saugbox (14) der Formkammer (12a, 12b) gegenübersteht und mit einem Unterdruck zum Erzeugen des Stroms (15) zum Saugen der Fasern aus der Formkammer (12a, 12b) in Richtung des luftdurchlässigen Mittels auf dem Formrad (13) bereitgestellt ist.

4. Vorrichtung gemäß Anspruch 3, bei der eine erste Saugbox (14) für jede Formkammer (12a, 12b) bereitgestellt ist.

5. Vorrichtung gemäß einem der vorstehenden Ansprüche, bei der das erste Material Zellstofffasern sind.

6. Vorrichtung gemäß einem der vorstehenden Ansprüche, bei der die Partikel des zweiten Materials Partikel oder Fasern eines hochabsorbierenden Polymers oder wärmeaktivierbaren Bindemittels sind.

7. Vorrichtung gemäß einem der vorstehenden Ansprüche, bei der das luftdurchlässige Mittel mindestens ein Element der folgenden Gruppe aufweist: ein Netz, eine mit Löchern bereitgestellte Platte, ein luftdurchlässiger Stoff.

8. Vorrichtung gemäß einem der vorstehenden Ansprüche, bei der eine nicht rotierende zweite Saugbox (20) innerhalb des Umfangs des Formrads (13) bereitgestellt ist, wobei die zweite Saugbox (20) entlang des zweiten Kreisbogens des Umfangs des Formrads (13) angeordnet ist und mit einem Unterdruck zum Ansaugen der gebildeten Lage oder der gebildeten Lagen gegen das luftdurchlässige Mittel auf der Fläche des Formrads (13) bereitgestellt ist.

9. Vorrichtung gemäß Anspruch 8, bei der das Band (21) angeordnet ist, um der zweiten Saugbox (20) gegenüber zu stehen.

10. Vorrichtung gemäß Anspruch 9, bei der die Saugbox (20) sich nur entlang eines Abschnitts des zweiten Kreisbogens des Formrads (13), das mit dem Band bedeckt ist, erstreckt.

11. Vorrichtung gemäß einem der vorstehenden Ansprüche, bei der das Band (21) ein Endlosband ist, das in einer geschlossenen Schleife läuft und mittels Stützrollen (22) unterstützt ist.

12. Vorrichtung gemäß Anspruch 11, bei der mindestens eine der Rollen (22) angeordnet ist, um eine Federkraft auf das Band (21) auszuüben, um das Band so zu dehnen, dass das Band (21) gegen das Formrad (13) mit einer festgelegten Kraft drückt.

13. Vorrichtung gemäß einem der Ansprüche 8 bis 12, bei der das Band (21) eine Breite aufweist, die breit genug ist, um die Formen (16) des Formrads (13) in einer lateralen Richtung zu bedecken.

14. Verwendung der Vorrichtung gemäß einem der vorstehenden Ansprüche zum Herstellen eines absorbierenden Kerns (C) in einem absorbierenden Produkt der Gruppe: Damenbinde, Windel, Trainingshose, Inkontinenz-Pad.

15. Verfahren zum Herstellen einer absorbierenden Struktur, die ein erstes Material enthält und ein zumindest innerhalb eines Abschnitts des ersten Materials verteiltes zweites Material, unter Verwendung der Vorrichtung gemäß einem der Ansprüche 1 bis 13, mit den Schritten:
- Bereitstellen eines Formrads (13), das ein luftdurchlässiges Mittel entlang seiner Umfangsfläche aufweist,
- Rotieren des Formrads (13),
- Anordnen mindestens einer Formkammer (12a, 12b) entlang eines ersten Kreisbogens der Fläche,
- Aufnehmen von Fasern des ersten Materials in der Formkammer (12a, 12b),
- Leiten der Fasern des ersten Materials in einem Strom (15) in Richtung der Fläche des Formrads (13),
- Einführen von Partikeln des zweiten Materials in den Strom (15) in mindestens einer der Formkammern (12a, 12b),
- Ablegen mindestens einer Lage des ersten und zweiten Materials auf der Fläche,
- Bereitstellen mindestens einer Form (16) entlang der Umfangsfläche,
- Bedecken der Form (16) entlang eines zweiten Kreisbogens des Formrads (13) mit einem Band (21), das im Wesentlichen mit der gleichen Geschwindigkeit wie das Formrad (13) an der Fläche des Formrads läuft.

16. Verfahren gemäß Anspruch 15, des Weiteren mit dem Schritt:
- Einrichten des Bands (21), um luftdurchlässig zu sein.

17. Verfahren gemäß Anspruch 15 oder 16, des Weiteren mit dem Schritt:
- Einrichten des Bands (21), um in einer geschlossenen Schleife zu laufen.

18. Verfahren gemäß einem der Ansprüche 15 bis 17, des Weiteren mit dem Schritt:
- Dehnen des Bands (21), sodass es einen Druck gegen die Lage oder die Lagen des ersten und zweiten Materials in der mindestens einen Form (16) auf der Fläche ausübt.

19. Verwendung der Vorrichtung gemäß einem der Ansprüche 1 bis 13 zum Herstellen eines absorbierenden Kerns (C) in einem absorbierenden Produkt, wobei der absorbierende Kern aus mehr als einer Lage besteht und die Vorrichtung eine Anzahl von Formrädern (13) für die Produktion mindestens einer Lage pro Formrad (13) aufweist und wobei jedes Formrad (13) mit dem Band (21) bereitgestellt ist.

## Revendications

1. Dispositif de fabrication d'un article absorbant contenant un premier matériau et un deuxième matériau dispersé dans au moins une partie dudit premier matériau, comprenant :
- une roue de formation rotative (13) munie d'un moyen perméable à l'air le long de sa surface périphérique,
- un moyen pour diriger au moins un mélange de fibres dudit premier matériau et de particules dudit deuxième matériau vers ladite surface de ladite roue de formation (13) pour déposer au moins une couche d'un mélange desdites fibres et desdites particules le long d'un premier arc de ladite surface de la roue de formation (13),
**caractérisé en ce que** :
une courroie perméable à l'air (21) est placée le long d'un deuxième arc de la surface périphérique de la roue de formation (13), dans lequel ladite courroie (21) porte sur ladite au moins une couche formée desdits premier et deuxième matériaux et va à la même vitesse que la surface de la roue de formation (13) pour maintenir en place les fibres et particules de la ou les couche(s) formée(s) sur la roue de formation (13),
dans lequel ledit moyen comprend :
- au moins une chambre de formation (12a, 12b) le long dudit premier arc de ladite surface pour recevoir au moins les fibres dudit premier matériau et un moyen d'aspiration pour diriger les fibres dudit premier matériau dans un courant (15) vers ladite surface de la roue de formation (13) pour déposer une couche desdites fibres sur la surface et au moins une desdites chambres de formation (12a, 12b) étant pourvue d'un mélange des fibres dudit premier matériau et des particules dudit deuxième matériau, et
dans lequel la surface périphérique de la roue de formation (13) est pourvue d'un moule continu (16) ou d'un nombre de moules distincts (16) le long de la surface.

2. Dispositif selon la revendication 1, dans lequel ladite au moins une chambre de formation (12) pourvue à la fois des fibres du premier matériau et des particules du deuxième matériau possède une entrée séparée (17) pour l'introduction des particules du deuxième matériau dans ledit courant (15).

3. Dispositif selon la revendication 1 ou 2, dans lequel ledit moyen d'aspiration pour diriger les fibres vers la roue de formation (13) comprend au moins une première boîte d'aspiration non rotative (14) à l'intérieur de la circonférence de la roue de formation (13), ladite au moins une boîte (14) faisant face à ladite chambre de formation (12a, 12b) et étant pourvue d'une dépression pour générer ledit courant (15) pour aspirer les fibres de la chambre de formation (12a, 12b) dans la direction du moyen perméable à l'air sur la roue de formation (13).

4. Dispositif selon la revendication 3, dans lequel une première boîte d'aspiration (14) est prévue pour chaque chambre de formation (12a, 12b).

5. Dispositif selon l'une quelconque des revendications précédentes, dans lequel le premier matériau est constitué de fibres de pâte à papier.

6. Dispositif selon l'une quelconque des revendications précédentes, dans lequel les particules du deuxième matériau sont des particules ou des fibres de polymère superabsorbant ou d'un liant activable à la chaleur.

7. Dispositif selon l'une quelconque des revendications précédentes, dans lequel ledit moyen perméable à l'air comprend au moins un élément de l'ensemble comprenant : un filet, une plaque pourvue de trous, une toile perméable à l'air.

8. Dispositif selon l'une quelconque des revendications précédentes, dans lequel une deuxième boîte d'aspiration non rotative (20) est placée à l'intérieur de la circonférence de la roue de formation (13), ladite deuxième boîte d'aspiration (20) étant placée le long dudit deuxième arc de la circonférence de la roue de formation (13) et étant pourvue d'une dépression pour aspirer la ou les couche(s) formée(s) contre le moyen perméable à l'air sur la surface de la roue de formation (13).

9. Dispositif selon la revendication 8, dans lequel ladite courroie (21) est agencée pour être en face de ladite deuxième boîte d'aspiration (20).

10. Dispositif selon la revendication 9, dans lequel ladite boîte d'aspiration (20) ne s'étend que sur une partie du deuxième arc de la roue de formation (13) couverte par la courroie.

11. Dispositif selon l'une quelconque des revendications précédentes, dans lequel ladite courroie (21) est une courroie sans fin tournant en boucle fermée et supportée au moyen de rouleaux de support (22).

12. Dispositif selon la revendication 11, dans lequel au moins l'un desdits rouleaux (22) est agencé pour exercer une force résiliente sur ladite courroie (21) pour étirer la courroie de telle manière que la courroie (21) exerce une pression contre la roue de formation (13) avec une force prédéterminée.

13. Dispositif selon l'une quelconque des revendications 8 à 12, dans lequel la courroie (21) a une largeur suffisamment grande pour couvrir les moules (16) de la roue de formation (13) dans une direction latérale.

14. Utilisation du dispositif selon l'une quelconque des revendications précédentes pour fabriquer un noyau absorbant (C) dans un article absorbant appartenant à l'ensemble comprenant : serviette hygiénique, couche, couche-culotte, protection contre l'incontinence.

15. Procédé de fabrication d'une structure absorbante contenant un premier matériau et un deuxième matériau dispersé dans au moins une partie dudit premier matériau, en utilisant le dispositif selon l'une quelconque des revendications 1 à 13, comprenant les étapes suivantes :
- fournir une roue de formation (13) comportant un moyen perméable à l'air le long de sa surface périphérique,
- faire tourner ladite roue de formation (13),
- agencer au moins une chambre de formation (12a, 12b) le long d'un premier arc de ladite surface,
- recevoir dans ladite chambre de formation (12a, 12b) des fibres dudit premier matériau,
- diriger lesdites fibres du premier matériau dans un courant (15) vers ladite surface de la roue de formation (13),
- introduire dans au moins l'une desdites chambres de formation (12a, 12b) des particules dudit deuxième matériau dans ledit courant (15),
- déposer au moins une couche desdits premier et deuxième matériaux sur ladite surface,
- placer au moins un moule (16) le long de ladite surface périphérique,
- couvrir ledit moule (16) le long d'un deuxième arc de la roue de formation (13) avec une courroie (21) allant sensiblement à la même vitesse que la roue de formation (13) à la surface de ladite roue.

16. Procédé selon la revendication 15, comprenant en outre l'étape suivante :
- adapter ladite courroie (21) pour qu'elle soit perméable à l'air.

17. Procédé selon la revendication 15 ou 16, comprenant en outre l'étape suivante :
- adapter ladite courroie (21) pour qu'elle tourne en boucle fermée.

18. Procédé selon l'une quelconque des revendications 15 à 17, comprenant en outre l'étape suivante :
- étirer ladite courroie (21) de manière telle qu'elle exerce une pression sur la ou les couches des premier et deuxième matériaux dans ledit au moins un moule (16) sur ladite surface.

19. Utilisation du dispositif selon l'une quelconque des revendications 1 à 13 pour la fabrication d'un noyau absorbant (C) dans un article absorbant, ledit noyau absorbant étant constitué de plus d'une couche, le dispositif comprenant un nombre de roues de formation (13) pour la production d'au moins une couche par roue de formation (13) et chaque roue de formation (13) étant pourvue de ladite courroie (21).
